# EUROPEAN PATENT APPLICATION

(11) **EP 2 918 268 A1**
(43) Date of publication of application: **16.09.2015**
(21) Application number: 15158167.5
(22) Date of filing: 09.03.2015
(51) Int. Cl.: A61K 9/48, A61K 31/00

(54) **Pharmaceutical compositions comprising an active agent**

(30) Priority: 10.03.2014 EP 14158478
(71) Applicant: Galenicum Health S.L., 08005 Barcelona (ES)
(72) Inventor: Arroyo Hidalgo, Sergio, 08005 Barcelona (ES); Puigvert Colomer, Marina, 08005 Barcelona (ES)
(74) Representative: Galenicum Health S.L.

(57) **Abstract**

The present invention relates to pharmaceutical compositions comprising the active agent racecadotril, to a process for the preparation thereof, and to the use thereof in the treatment of diarrhoea.

## Description

The present invention relates to pharmaceutical compositions comprising the active agent racecadotril, to a process for the preparation thereof, and to the use thereof in the treatment of diarrhoea.

### STATE OF THE ART

(RS)-benzyl N-[3-(acetylthio)-2-benzylpropanoyl]glycinate is also known as benzyl (RS)-2-[[2-[(acetylsulphanyl)methyl]-3-phenylpropanoyl]amino]acetate, racecadotril or acetorphan, its empirical formula is C21H23NO4S and the compound has a molecular weight of 385.48 g/mol. Racecadotril is the compound of structure (I) in the form of a racemate:

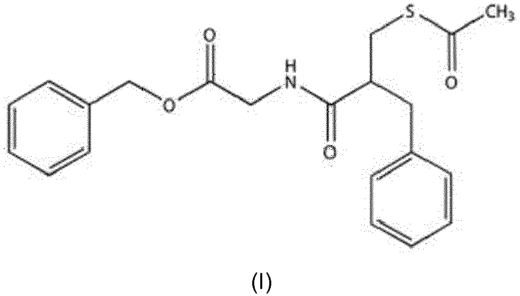

Racecadotril is an antidiarrheal drug which acts as a peripherally acting enkephalinase inhibitor. Unlike other medications used to treat diarrhea, which reduce intestinal motility, racecadotril has an antisecretory effect, reducing the secretion of water and electrolytes into the intestine.

Hidrasec® and Tiorfan® are medicinal products indicated for the symptomatic treatment of acute diarrhoea in adults when causal treatment is not possible. If causal treatment is possible, racecadotril can be administered as a complementary treatment. These products contain racecadotril as the active agent and are presented as hard capsules containing 100 mg of the active agent in the form of a powder.

Racecadotril was first disclosed in EP0038758. WO01/97803, EP1294372, US2009/0186084 and EP2462922 disclose oral formulations comprising said active agent.

### DESCRIPTION OF THE INVENTION

The present invention provides a stable pharmaceutical composition comprising racecadotril as active agent which have a low impurity content and can be easily manufactured.

The granulates of the present invention can be easily manufactured and filled in capsules, since they show excellent flow properties. Moreover, said granulates show an improved stability both encapsulated and nonencapsulated, especially when magnesium stearate is used as extragranular lubricant. Also, the extragranular lubricant entails the desired dissolution profile of at least 60 % of the active agent of the pharmaceutical composition being dissolved in 30 minutes in a basket dissolution test apparatus, at 100 rpm with a 3 % w/v sodium lauryl sulfate solution in water as dissolution medium and a volume of 900 ml per vessel. The inventors have surprisingly found that stable granulates comprising racecadotril can be produced by a simple dry granulation method when the granulates comprise a hydrophilic fumed silica having a BET surface area between 140 and 265 m²/g.

The term "stable" as used herein refers to a pharmaceutical composition comprising racecadotril wherein the total content of impurities originating from the decomposition of racecadotril does not exceed 5 % area, preferably 3 % area, more preferably 2 % area and most preferably 1 % area determined by liquid chromatography (HPLC) at 230 nm if such a composition is stored for 1 month at 40 °C and 75 % relative humidity (RH).

In a first aspect, the present invention relates to a pharmaceutical composition comprising a granulate and an extragranular lubricant, wherein said granulate comprises the active agent racecadotril, and wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel.

The term "pharmaceutical granulate" or "granulate" or "granular component" as used herein refers to the part of the pharmaceutical composition that has been obtained by granulating a powder into larger particles herein called granules. As used herein, the term "compacted granulate" refers to a granulate that has been obtained by dry granulation, i.e: by compacting a pharmaceutical powder and then milling and sieving the slug or laminate obtained by compaction. The dry granulation is performed for example by slugging or using a roller compactor. As used herein, the term "pharmaceutical powder" refers to an intimate mixture of dry particles of an active agent and at least one pharmaceutically acceptable excipient or inactive ingredient.

The term "extragranular component" as used herein refers to the set of ingredients in the pharmaceutical composition which have not been granulated and which do not form part of the granulate or granular component.

As used herein, "lubricant" means a substance that reduces the friction both between the components of the composition of the present invention, and between the composition and the surfaces of the apparatus used to prepare said composition.

In a preferred embodiment, the lubricant is selected from stearic acid, magnesium stearate, polyethylene glycol, sodium stearyl fumarate, sodium benzoate and mixtures thereof.

In a preferred embodiment, the lubricant is selected from magnesium stearate, sodium stearyl fumarate and mixtures thereof. In a more preferred embodiment, the lubricant is magnesium stearate. In another preferred embodiment, the lubricant is sodium stearyl fumarate. In a preferred embodiment, when the lubricant is magnesium stearate, said lubricant is exclusively extragranular.

In a preferred embodiment, the granulate comprises a hydrophilic colloidal silicon dioxide having a BET surface area between 140 and 265 m²/g. Preferably, the granulate comprises a hydrophilic colloidal silicon dioxide having a BET surface area between 150 and 250 m²/g. More preferably, the granulate comprises a hydrophilic colloidal silicon dioxide having a BET surface area between 175 and 225 m²/g. The BET surface area is calculated according to according to the European Pharmacopoeia (2.9.26). The pharmaceutical compositions of the present invention have the best stability results when they comprise hydrophilic colloidal silicon dioxide having a BET surface area between 175 and 225 m²/g.

In a preferred embodiment, the weight ratio active agent:hydrophilic colloidal silicon dioxide is between 4:1 and 200:1. Preferably, the weight ratio active agent:hydrophilic colloidal silicon dioxide is between 5:1 and 150:1. More preferably, the weight ratio active agent:hydrophilic colloidal silicon dioxide is between 20:1 and 100:1.

In a preferred embodiment, the pharmaceutical composition contains an amount of active agent per unit dose between 9 and 110 mg. In a preferred embodiment, the pharmaceutical composition contains an amount of active agent per unit dose of between 9.5 and 10.5 mg, or between 28.5 and 31.5 mg, or between 95 and 105 mg. In a preferred embodiment, the pharmaceutical composition contains an amount of active agent per unit dose of between 95 and 105 mg.

As used herein, the term "unit dose" or "unit dosage" refers to a physically discrete unit that contains a predetermined quantity of active agent calculated to produce a desired therapeutic effect. The unit dose or unit dosage may be in the form of a vial, tablet, capsule, sachet, etc. referred to herein as a "unit dosage form".

In a preferred embodiment, the particle size volume distribution of the active agent has a D50 between 1 and 10 microns and/or a D90 between 12 and 40 microns, when measured by laser diffraction analysis. In a preferred embodiment, the particle size volume distribution of the active agent has a D50 between 2 and 9 microns and/or a D90 between 14 and 35 microns, when measured by laser diffraction analysis. In a preferred embodiment, the particle size volume distribution of the active agent has a D50 between 3 and 8 microns and/or a D90 between 16 and 30 microns, when measured by laser diffraction analysis.

In a preferred embodiment, the granulate is a compressed granulate. A compressed granulate is a granulate obtained by dry granulation.

In a preferred embodiment, the particle size volume distribution of the granulate has a D50 between 20 and 300 microns and/or a D90 between 350 and 800 microns, when measured by laser diffraction analysis. In a preferred embodiment, the particle size volume distribution of the granulate has a D50 between 50 and 250 microns and/or a D90 between 380 and 720 microns, when measured by laser diffraction analysis. In a preferred embodiment, the particle size volume distribution of the granulate has a D50 between 80 and 200 microns and/or a D90 between 400 and 700 microns, when measured by laser diffraction analysis.

In a preferred embodiment, the pharmaceutical composition further comprises at least an acid, preferably the acid is intragranular. In a preferred embodiment, the acid is selected from tartaric acid, citric acid and ascorbic acid. In a preferred embodiment, the acid is citric acid, preferably anhydrous citric acid.

In a preferred embodiment, the pharmaceutical composition comprises a diluent selected from microcrystalline cellulose, dicalcium phosphate, sorbitol, lactose and mixtures thereof. In a preferred embodiment, the diluent is selected from microcrystalline cellulose, lactose and mixtures thereof. In a preferred embodiment, the diluent is lactose monohydrate.

The term "diluent" as used herein refers to pharmaceutically acceptable excipients which are added to the bulk volume of the active agent making up the solid composition. As a result, the size of the solid composition increases, which makes its size suitable for handling. Diluents are convenient when the dose of drug per solid composition is low and the solid composition would otherwise be too small.

The term "pharmaceutically acceptable" indicates that the substance or composition must be compatible chemically and/or toxicologically, with the other ingredients comprising a formulation, the mammal being treated therewith, and/or the route of administration of the composition.

In a preferred embodiment, the pharmaceutical composition comprises a disintegrant selected from starch, pregelatinized starch, sodium carboxymethyl cellulose, croscarmellose sodium, crosslinked polyvinylpyrrolidone, low-substituted hydroxypropyl cellulose and mixtures thereof. Preferably, the disintegrant is selected from starch, pregelatinised maize starch and mixtures thereof. More preferably, the disintegrant is pregelatinised maize starch.

In a preferred embodiment, the granulate is manufactured by dry granulation.

In a preferred embodiment, the pharmaceutical composition is in the form of tablets, capsules, sachets or liquid suspension, preferably in the form of capsules, more preferably hard capsules.

A preferred embodiment of the present invention relates to a pharmaceutical composition comprising a granulate and an extragranular lubricant, wherein said granulate comprises the active agent racecadotril, and wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel, wherein the lubricant is magnesium stearate and wherein the granulate further comprises colloidal silicon dioxide. Preferably, the colloidal silicon dioxide is exclusively intragranular and is hydrophilic.

A second aspect of the present invention relates to a hard capsule comprising the pharmaceutical composition of the first aspect. Preferably, said capsule is a hard gelatine capsule.

A third aspect of the present invention relates to a sachet comprising the pharmaceutical composition of the first aspect.

A fourth aspect of the present invention relates to a stick-pack comprising the pharmaceutical composition of the first aspect.

The term "sachet" or "stick pack" as used herein refers to a small, sealed packet containing a quantity of material, which is a single use or unit dose quantity. A packaging of the stick pack type, preferably with improved opening, comprises normally: a flexible film, having at least one layer, which forms a hermetically sealed tubular body with mutually opposite longitudinal film flaps, a first band provided longitudinally to said body for inside/outside sealing of said mutually opposite longitudinal flaps of the film; second sealing bands provided transversely to said body for inside/outside sealing; a sealed extension region protruding from at least one of said second sealing bands on a respective portion of at least one edge of said tubular body; and preferably a transverse pre-weakening incisions that are provided in longitudinal alignment with said sealed extension region, along at least one of said mutually opposite longitudinal flaps. Suitable stick packs are described in WO9501921.

A fifth aspect of the present invention relates to a pharmaceutical batch comprising at least 20,000 units of the pharmaceutical composition of the first, second, third or fourth aspect. Preferably, the batch comprises at least 50,000 units of said pharmaceutical composition.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active agent in the compositions of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active agent" refers to a therapeutically active compound, as well as any prodrugs thereof and pharmaceutically acceptable salts, hydrates and solvates of the compound and the prodrugs.

For the release of a pharmaceutical batch the distribution of the active agent in the pharmaceutical compositions has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

A sixth aspect of the present invention relates to a process for the manufacture of a pharmaceutical composition comprising a granulate and an extragranular lubricant, wherein said granulate comprises the active agent racecadotril, and wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel; wherein the process comprises the following steps:
(i) mixing the active agent racecadotril with at least one pharmaceutically acceptable excipient;
(ii) granulating the mix obtained in step (i); and
(iii) adding at least one extragranular lubricant to the granules obtained in step (ii).

In a preferred embodiment, the granulation of step (ii) is a dry granulation comprising compacting, milling and sieving the mix obtained in step (i) to form granules. In a preferred embodiment, step (i) comprises mixing the active agent racecadotril with a hydrophilic colloidal silicon dioxide having a BET surface area between 140 and 265 m²/g, wherein the weight ratio active agent:hydrophilic colloidal silicon dioxide is between 4:1 and 200:1. In a preferred embodiment, the process is carried out under red light.

A seventh aspect of the present invention relates to the pharmaceutical composition obtained by the process of the sixth aspect.

An eighth aspect of the present invention relates to the pharmaceutical composition of the first or seventh aspect, the hard capsule of the second aspect, the sachet of the third aspect, the stick-pack of the fourth aspect, or the pharmaceutical batch of the fifth aspect, for use in the treatment of diarrhoea. Preferably, for use in the treatment of acute diarrhoea in adults when causal treatment is not possible or as a complementary treatment when causal treatment is possible.

A ninth aspect of the present invention relates to a blister pack comprising at least one unit of the pharmaceutical composition of the first, second, third, fourth or seventh aspect, wherein said blister pack is preferably an aluminum/PVC blister, an aluminum/aluminum blister, or a PVC//PVDC/aluminum blister. Preferably, said blister is a PVC//PVDC/aluminum blister.

The term "blister" or bubble pack refers to a sheet in a package construction with recesses designed to hold dosage forms. The sheet may be a plastic, a foil, or combination thereof. Normally, a blister is a product consisting of a flat structure in which blisters are formed, generally by means of a heating process, into which the single elements to be packaged are inserted. The blisters are then hermetically sealed using flat strips of appropriate thermomoldable materials (plastics, aluminum, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminium/PVC blister refers to a blister the thermomoldable material is from PVC and the backing is a lidding seal of aluminium foil. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit- dose blister packs over other methods of packing pharmaceutical products are the assurance of product/packaging integrity (including shelf life) of each individual dose and the possibility to create a compliance pack or calendar pack by printing the days of the week above each dose. Blister packs can be created by means of a form-fill- seal process at the pharmaceutical company or designated contract packer. A form- fill-seal process means that the blister pack is created from rolls of flat sheet or film, filled with the pharmaceutical product and closed (sealed) on the same equipment. Such equipment is called a blister line. There are two types of blister machine's design: rotary and flat-plate.

A tenth aspect of the present invention relates to a cardboard box with a patient information leaflet comprising at least one aluminum/PVC or PVC/PVDC/aluminum blister pack of at least 3 units of the pharmaceutical composition of the first, second or seventh aspect.

An eleventh aspect of the present invention relates to a cardboard box with a patient information leaflet comprising at least one sachet of the third aspect or at least one stick-pack of the fourth aspect.

In a preferred embodiment, the present invention relates to a pharmaceutical composition comprising a granulate and extragranular sodium stearyl fumarate, wherein said granulate comprises the active agent racecadotril, wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel, wherein the granulate further comprises a hydrophilic colloidal silicon dioxide having a BET surface area between 175 and 225 m²/g, wherein the weight ratio active agent:hydrophilic colloidal silicon dioxide is between 20:1 and 100:1, wherein the pharmaceutical composition contains an amount of active agent per unit dose of between 95 and 105 mg and wherein the particle size volume distribution of the active agent has a D50 between 1 and 10 microns and/or a D90 between 12 and 40 microns, when measured by laser diffraction analysis.

In a preferred embodiment, the present invention relates to a pharmaceutical composition in form of a hard capsule comprising a granulate and extragranular magnesium stearate, wherein said granulate comprises the active agent racecadotril, wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel, wherein the magnesium stearate is exclusively extragranular, wherein the granulate comprises a hydrophilic colloidal silicon dioxide having a BET surface area between 140 and 265 m²/g, wherein the granulate is a compressed granulate manufactured by dry granulation, wherein the particle size volume distribution of the granulate has a D50 between 50 and 250 microns and/or a D90 between 380 and 720 microns, when measured by laser diffraction analysis, wherein the pharmaceutical composition comprises lactose monohydrate as diluent and pregelatinised maize starch as disintegrant. Preferably, said composition contains an amount of active agent per unit dose of between 95 and 105 mg and the particle size volume distribution of the active agent has a D50 between 1 and 10 microns and/or a D90 between 12 and 40 microns, when measured by laser diffraction analysis.

All percentages, parts, and ratios herein are by weight unless specifically noted otherwise. As used herein, the term "about" refers preferably to a range that is ±10 %, preferably ±5 %, or more preferably ±1 % of a value with which the term is associated.

Unless otherwise indicated, all the analysis methods are carried out according to the European Pharmacopoeia 7th edition.

### EXAMPLES

### Example 1. Pharmaceutical compositions.

| *Intragranular* | Ex. 1.10 | Ex. 1.12 | Ex. 1.13.16 | Ex. 1.14 | Ex. 1.15 |
|---|---|---|---|---|---|
| Active agent | 100.0 | 100.0 | 100.0 | 100.0 | 100.0 |
| Lactose monohydrate | 27.0 | 36.0 | 41.0 | 41.0 | 41.0 |
| Pregelatinised corn starch | 93.0 | 84.8 | 78.3 | 78.3 | 78.0 |
| Hydrophilic colloidal silica | 1.0 | 2.0 | 4.5 | 4.5 | - |
| Sodium stearyl fumarate | 2.0 | 1.0 | - | - | - |
| Citric acid | - | 0.2 | - | - | - |
| Hydrophobic colloidal silica | - | - | - | - | 2.0 |
| *Extragranular* | | | | | |
| Magnesium stearate | - | - | - | 1.2 | 4.0 |
| Hydrophobic colloidal silica | 1.0 | - | - | - | - |
| Sodium stearyl fumarate | 1.0 | 1.0 | 1.2 | - | - |
| Total (g) | 225.0 | 225.0 | 225.0 | 225.0 | 225.0 |

The intragranular ingredients were mixed, and the mix was compressed, milled and sieved to form granules. The extragranular ingredients were then added and mixed with the granules. Hard capsules were filled with 225 mg per capsule. The capsules were packaged in PVC/PVDC/Alu blisters.

### Example 2: Assay and Stability.

Capsules of example 1 were subjected to stability tests at times 0, and 1 month at 25 °C and 60 % Relative Humidity (RH), at 30 °C and 65 % RH, and at 40 °C and 75 % RH.

The active agent content and the amount of impurities in the capsules were analysed by HPLC. The area corresponding to the active agent and of the major peaks, if any, was determined. The percentage of impurities was calculated by comparing the areas of the measured peaks with those obtained from the standard.

**Table 1. Assay.**

| Example | t=0 | t=1 month, 25 °C and 60 % RH | t=1 month, 30 °C and 65 % RH | t=1 month, 40 °C and 75 % RH |
|---|---|---|---|---|
| 1.10 | 100.5 | 102.9 | 101.2 | 98.0 |
| 1.12 | 98.7 | 98.4 | 98.0 | 99.8 |
| 1.13.16 | 99.6 | 102.1 | 98.8 | 101.2 |
| 1.14 | 103.6 | 97.7 | 98.7 | 101.1 |
| 1.15 | 102.7 | N/A | N/A | N/A |

**Table 2. Percentage of total impurities.**

| Example | t=0 | t=1 month, 25 °C and 60 % RH | t=1 month, 30 °C and 65 % RH | t=1 month, 40 °C and 75 % RH |
|---|---|---|---|---|
| 1.10 | 0.03 | 0.06 | 0.04 | 0.06 |
| 1.12 | 0.01 | 0.04 | 0.06 | 0.08 |
| 1.13.16 | 0.03 | 0.04 | 0.04 | 0.09 |
| 1.14 | 0.05 | 0.16 | 0.16 | 0.19 |
| 1.15 | 0.21 | N/A | N/A | N/A |

### Example 3: Water content.

The amount of water of the pharmaceutical compositions as herein disclosed was measured by loss on drying (LOD) using a Halogen Moisture Analyzer.

**Table 3. Water content (% w/w):**

| Example | t=0 | t=1 month, 25 °C and 60 % RH | t=1 month, 30 °C and 65 % RH | t=1 month, 40 °C and 75 % RH |
|---|---|---|---|---|
| 1.10 | 3.9 | 3.7 | 4.1 | 5.3 |
| 1.12 | 3.0 | 3.6 | 3.7 | 4.4 |
| 1.13.16 | 1.9 | 2.1 | 2.6 | 3.1 |
| 1.14 | 2.5 | 2.3 | 3.0 | 3.9 |

### Example 4: Particle size volume distribution.

The particle size distribution of the active agent and of the granulate was analysed by laser diffraction spectroscopy using a Malvern Mastersizer 2000 particle size analyzer.

### Example 5. Dissolution profiles.

The dissolution profiles were analysed in the following conditions: USP Apparatus 1 (basket); speed: 100 rpm; medium: 3 % w/v sodium lauryl sulfate in water; volume: 900 ml per vessel; wavelength: 230 nm. The dissolution profiles of the capsules of the examples above are homogeneous and stable, and show more than 60 % of the active agent dissolved after 30 minutes. For example, the capsules of example 1 a show the following dissolution values:

| time (min) | Percentage active agent dissolved |
|---|---|
| 0 | 0 |
| 5 | 12 |
| 10 | 40 |
| 15 | 58 |
| 20 | 69 |
| 30 | 86 |
| 45 | 98 |
| 60 | 103 |

## Claims

1. A pharmaceutical composition comprising a granulate and an extragranular lubricant, wherein said granulate comprises the active agent racecadotril, and wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel.

2. The pharmaceutical composition according to the preceding claim, wherein the lubricant is selected from stearic acid, magnesium stearate, polyethylene glycol, sodium stearyl fumarate, sodium benzoate and mixtures thereof, preferably the lubricant is magnesium stearate or sodium stearyl fumarate, more preferably when the lubricant is magnesium stearate, said lubricant is exclusively extragranular.

3. The pharmaceutical composition according to any one of the preceding claims, wherein the granulate comprises a hydrophilic colloidal silicon dioxide having a BET surface area between 150 and 250 m²/g.

4. The pharmaceutical composition according to any one of the preceding claims, wherein the weight ratio active agent:hydrophilic colloidal silicon dioxide is between 5:1 and 150:1.

5. The pharmaceutical composition according to any one of the preceding claims, wherein the pharmaceutical composition contains an amount of active agent per unit dose of between 9.5 and 10.5 mg, or between 28.5 and 31.5 mg, or between 95 and 105 mg, preferably of between 95 and 105 mg; and/or wherein the particle size volume distribution of the active agent has a D50 between 2 and 9 microns and/or a D90 between 14 and 35 microns, when measured by laser diffraction analysis.

6. The pharmaceutical composition according to any one of the preceding claims, wherein the granulate is a compressed granulate; and/or wherein the particle size volume distribution of the granulate has a D50 between 50 and 250 microns and/or a D90 between 380 and 720 microns, when measured by laser diffraction analysis.

7. The pharmaceutical composition according to any one of the preceding claims, further comprising at least an acid, preferably the acid is intragranular, more preferably the acid is selected from tartaric acid, citric acid and ascorbic acid.

8. The pharmaceutical composition according to any one of the preceding claims, comprising: a diluent selected from microcrystalline cellulose, dicalcium phosphate, sorbitol, lactose and mixtures thereof, preferably the diluent is lactose monohydrate; and/or comprising a disintegrant selected from starch, pregelatinized starch, sodium carboxymethyl cellulose, croscarmellose sodium, crosslinked polyvinylpyrrolidone, low-substituted hydroxypropyl cellulose and mixtures thereof, preferably the disintegrant is pregelatinised maize starch.

9. The pharmaceutical composition according to any one of the preceding claims, wherein the granulate is manufactured by dry granulation.

10. A tablet, a capsule, preferably a hard capsule, more preferably a hard gelatin capsule, a sachet, a stick-pack or a liquid suspension comprising the pharmaceutical composition as defined in any one of claims 1 to 9.

11. A pharmaceutical batch comprising at least 20,000 units, preferably 50,000 units of the pharmaceutical composition according to any one of the preceding claims.

12. A process for the manufacture of a pharmaceutical composition comprising a granulate and an extragranular lubricant, wherein said granulate comprises the active agent racecadotril, and wherein at least 60 % of the active agent of the pharmaceutical composition dissolves in 30 minutes in a basket dissolution test apparatus, at 100 rpm with a 3 % w/v sodium lauryl sulfate solution in water as dissolution medium and a volume of 900 ml per vessel; wherein the process comprises the following steps, and wherein said steps are preferably carried out under red light:
(i) mixing the active agent racecadotril with at least one pharmaceutically acceptable excipient, preferably with a hydrophilic colloidal silicon dioxide having a BET surface area between 140 and 265 m²/g, wherein the weight ratio active agent:hydrophilic colloidal silicon dioxide is between 4:1 and 200:1;
(ii) granulating the mix obtained in step (i), preferably by dry granulation comprising compacting, milling and sieving the mix obtained in step (i); and
(iii) adding at least one extragranular lubricant to the granules obtained in step (ii).

13. The pharmaceutical composition obtained by the process as described in the preceding claim.

14. The pharmaceutical composition according to any one of claims 1 to 9 or 13, the hard capsule, the sachet or the stick-pack according to claim 10, or the pharmaceutical batch according claim 11, for use in the treatment of diarrhoea.

15. A cardboard box with a patient information leaflet comprising at least one aluminum/PVC or PVC/PVDC/aluminum blister pack of at least 3 units of the pharmaceutical composition as defined in any one of claims 1 to 9 or in claim 13 or of the capsule according to claim 10, or comprising at least one sachet or at least one stick-pack comprising the pharmaceutical composition as defined in any one of claims 1 to 9 or in claim 13.
